# EUROPEAN PATENT APPLICATION

(11) **EP 2 458 518 A2**
(43) Date of publication of application: **30.05.2012**
(21) Application number: 11189086.9
(22) Date of filing: 15.11.2011
(51) Int. Cl.: G06F 19/00

(54) **Remote healthcare system and healthcare method using the same**

(30) Priority: 26.11.2010 KR 20100118593
(71) Applicant: Samsung Electronics Co., Ltd., Suwon-si, Gyeonggi-do 442-742 (KR)
(72) Inventor: Lee, Sung Hwa, Gyeonggi-Do (KR); Kim, Dong Woo, Gyeonggi-do (KR); Kim, Se Un, Seoul (KR); Park, Ji Eun, Seoul (KR); Yoon, Ji Na, Seoul (KR)
(74) Representative: Land, Addick Adrianus Gosling

(57) **Abstract**

A remote healthcare system and healthcare method using the same are provided. A healthcare method includes accessing a healthcare system, acquiring vital information of a user who accesses the healthcare system, and displaying a progress state of a healthcare service and a health of the user as an image based on the vital information.

## Description

The following description relates to a remote healthcare system using a patient terminal or the Internet and a healthcare method using the same.

A remote medical service system, called, for example, a home healthcare system or a U-healthcare system, measures vital information of a patient using various measurement apparatuses at home without a visit to a hospital, thereby enabling the patient to remotely receive medical services based on the measurement result.

A gateway, which is, for example, a patient terminal used in a remote medical service, serves to collect vital information of a patient using various measurement apparatuses and transmit the vital information to a server of a hospital or a server of a healthcare center. Such a gateway is mainly used by elderly patients, such as, for example, chronic patients or cancer patients who require long-term healthcare. Accordingly, in order for the patients to successfully obtain benefit from the long-term healthcare, continuous vital information measurement is necessary.

In gateways of the related art, a user interface is designed so as to be easily used, in consideration of the fact that a gateway is used by elderly patients. However, since a healthcare service is generally used for a long period of time, a user interface that considers only ease of use does not motivate a patient to continue using the healthcare service. Since a user interface does not exist that enables a user to easily recognize that a patient's condition has improved by continuous healthcare, a motive for healthcare is lost. As a result, the patient may decide to no longer use the healthcare service.

In one general aspect, there is provided a healthcare method, including accessing a healthcare system, acquiring vital information of a user who accesses the healthcare system, and displaying a progress state of a healthcare service and a health of the user as an image based on the vital information.

The general aspect of the method may further provide that the healthcare system includes a gateway that is a terminal used to access the healthcare system, a vital information measurement sensor configured to measure the vital information of the user, and transmit the vital information to the gateway, and a server including a user database.

The general aspect of the method may further provide that the user database includes personal information, diagnostic information, healthcare items, measured vital information of the user, and a result of one or more analyses of the measured vital information of the user.

The general aspect of the method may further provide that the accessing of the healthcare system includes accessing the gateway, transmitting user account information from the gateway to the server, comparing the user account information with information stored in the user database at the server, and allowing access to the healthcare system if the user account information matches the information stored in the user database.

The general aspect of the method may further provide that the acquiring of the vital information includes collecting the vital information of the user using the vital information measurement sensor, transmitting the vital information to the gateway, and transmitting the vital information from the gateway to the server.

The general aspect of the method may further provide that the displaying of the progress includes determining the progress state of the healthcare service, determining the health of the user, displaying an image corresponding to the progress state of the healthcare service, and displaying an image corresponding to the health of the user.

The general aspect of the method may further provide that the determining of the progress state includes determining a number of times of measurement of the vital information, determining a time when the measurement of the vital information is performed, determining whether the number of times of measurement of the vital information corresponds to a predetermined number of times of measurement of the healthcare service, and determining whether the time when the vital information measurement is performed corresponds to a scheduled measurement time of the healthcare service.

The general aspect of the method may further provide that the determining of the health includes determining a change in the health of the user by comparing the vital information with previously measured vital information.

The general aspect of the method may further provide that the healthcare system includes a gateway that is a terminal used to access the healthcare system, a vital information measurement sensor configured to measure the vital information of the user, and transmit the vital information to the gateway, and a server including a user database, and the displaying of the image corresponding to the progress state of the healthcare service includes selecting an image corresponding to a current progress state of the healthcare service from among a plurality of different images corresponding to a change in the progress state of the healthcare service, and displaying the selected image on the gateway.

The general aspect of the method may further provide that the displaying of the image corresponding to the progress state of the healthcare service further includes displaying, on the gateway along with the image, an explanation of a progress rate of the healthcare service or the image.

The general aspect of the method may further provide that the healthcare system includes a gateway that is a terminal used to access the healthcare system, a vital information measurement sensor configured to measure the vital information of the user, and transmit the vital information to the gateway, and a server including a user database, and the displaying of the image corresponding to the health of the user includes selecting an image corresponding to a current health of the user from among a plurality of different images according to a change in the health of the user, and displaying the selected image on the gateway.

The general aspect of the method may further provide that the displaying of the image corresponding to the health of the user further includes displaying, on the gateway along with the image, an explanation of a progress rate of the healthcare service or the image.

The general aspect of the method may further provide that the healthcare system includes a server including a user database, a vital information measurement sensor configured to measure the vital information of the user, and transmit the vital information to the server, and a website used to access the healthcare system.

The general aspect of the method may further provide that the user database includes personal information, diagnostic information, healthcare items, measured vital information of the user, and a result of one or more analyses of the measured vital information of the user.

The general aspect of the method may further provide that the accessing of the healthcare system includes accessing the website, transmitting user account information from the website to the server, comparing the user account information with information stored in the user database at the server, and allowing access to the healthcare system if the user account information matches the information stored in the user database.

The general aspect of the method may further provide that the acquiring of the vital information includes collecting the vital information of the user using the vital information measurement sensor, transmitting the vital information to the server, and transmitting the vital information from the server to the website.

The general aspect of the method may further provide that the healthcare system includes a server including a user database, a vital information measurement sensor configured to measure the vital information of the user, and transmit the vital information to the server, and a website used to access the healthcare system, and the displaying of the image corresponding to the progress state of the healthcare service includes selecting an image corresponding to a current progress state of the healthcare service from among a plurality of different images corresponding to a change in the progress state of the healthcare service, and displaying the selected image on the website.

The general aspect of the method may further provide that the displaying of the image corresponding to the progress state of the healthcare service further includes displaying, on the website along with the image, an explanation of a progress rate of the healthcare service or the image.

The general aspect of the method may further provide that the healthcare system includes a server including a user database, a vital information measurement sensor configured to measure the vital information of the user, and transmit the vital information to the server, and a website used to access the healthcare system, and the displaying of the image corresponding to the health of the user includes selecting an image corresponding to a current health of the user from among a plurality of different images according to a change in the health of the user, and displaying the selected image on the website.

The general aspect of the method may further provide that the displaying of the image corresponding to the health of the user further comprises displaying, on the website along with the image, an explanation of a progress rate of the healthcare service or the image.

In another aspect, there is provided a gateway, including a communication unit configured to perform communication with a vital information measurement sensor and a server, a display unit configured to display an image corresponding to a progress state of a healthcare service and an image corresponding to a health of a user, and a controller configured to receive vital information from the vital information measurement sensor, determine the progress state of the healthcare service and the health of the user from the vital information, and display the image corresponding to the progress state of the healthcare service and the image corresponding to the health of the user on the display unit.

The general aspect of the gateway may further provide that the display unit operates in a touch screen manner.

The general aspect of the gateway may further provide that the image corresponding to the progress state of the healthcare service includes an image corresponding to a current progress state of the healthcare service among a plurality of different images according to a change in the progress state of the healthcare service.

The general aspect of the gateway may further provide that the controller is further configured to display, on the display unit along with the image corresponding to the progress state of the healthcare service, an explanation of a progress rate of the healthcare service or the image.

The general aspect of the gateway may further provide that the image corresponding to the health of the user includes an image corresponding to a current health of the user among a plurality of different images according to a change in the health of the user.

The general aspect of the gateway may further provide that the controller is further configured to display, along with the image corresponding to the health of the user, an explanation of a change in the health of the user or the image on the display unit.

The general aspect of the gateway may further provide that the healthcare service is determined according to the health of the user and comprises the vital information to be measured by the user, a number of times that the vital information has been measured, a measurement schedule of the vital information, a method of measuring the vital information, a kind of a drug to be taken, an amount of the drug that needs to be taken per unit time, when the drug is taken, a method of taking the drug, a menu, and an exercise plan of the user.

In another aspect, there is provided a healthcare system, including a server including a user database, a vital information measurement sensor configured to measure vital information of a user, and a website configured to display a progress state of a healthcare service and a health of the user based on the vital information of the user.

The general aspect of the healthcare system may further provide gateway configured to receive the vital information of the user from the vital information measurement sensor, and transmit the vital information to the server.

The general aspect of the healthcare system may further provide that the user database includes personal information, diagnostic information, healthcare items, measured vital information, and a result of one or more analyses of the measured vital information of the user.

The general aspect of the healthcare system may further provide that the website is further configured to determine a number of times of measurement of the vital information of the user, and a time when the vital information measurement is performed.

The general aspect of the healthcare system may further provide that the website is further configured to determine whether the number of times of measurement of the vital information corresponds to a predetermined number of times of measurement of the healthcare service, and whether the time when the vital information measurement is performed corresponds to a scheduled measurement time of the healthcare service.

The general aspect of the healthcare system may further provide that the website is further configured to display an image corresponding to a current progress state of the healthcare service among a plurality of different images according to a change in the progress state of the healthcare service so as to enable the user to recognize the progress state of the healthcare service.

The general aspect of the healthcare system may further provide that the website is further configured to analyze the vital information of the user, compare the analyzed result with a previously analyzed result stored in the server, and determine a change in the health of the user.

The general aspect of the healthcare system may further provide that the website is further configured to select and display an image corresponding to a current health of the user from among a plurality of different images according to a change in the health of the user.

In another aspect, there is provided a healthcare method, including receiving access information of a user who accesses a healthcare system, acquiring vital information of the user, and displaying a progress state of a healthcare service and a health of the user as an image based on the vital information.

Other features and aspects may be apparent from the following detailed description, the drawings, and the claims.

### BRIEF DESCRIPTION OF THE DRAWINGS

FIG. 1 is a block diagram illustrating an example of a remote healthcare system including a gateway.
FIG. 2 is a flowchart illustrating an example of a process of accessing a healthcare system including a gateway.
FIG. 3 is a flowchart illustrating an example of a process of acquiring vital information of a user.
FIGS. 4A and 4B are flowcharts illustrating examples of a process of displaying a progress state of a healthcare service and a health of a user as an image.
FIGS. 5A to 5C are conceptual diagrams illustrating examples of an image which changes according to the progress state of a healthcare service.
FIGS. 6A to 6C are conceptual diagrams illustrating examples of an image which changes according to user's health.
FIG. 7 is a block diagram illustrating an example of a remote healthcare system including a website.
FIG. 8 is a flowchart illustrating an example of a process of accessing a healthcare system including a website.
FIG. 9 is a flowchart illustrating another example of a process of acquiring vital information of a user.
FIG. 10 is a block diagram illustrating another example of a remote healthcare system.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following detailed description is provided to assist the reader in gaining a comprehensive understanding of the methods, apparatuses, and/or systems described herein. Accordingly, various changes, modifications, and equivalents of the systems, apparatuses and/or methods described herein will be suggested to those of ordinary skill in the art. Also, descriptions of well-known functions and constructions may be omitted for increased clarity and conciseness.

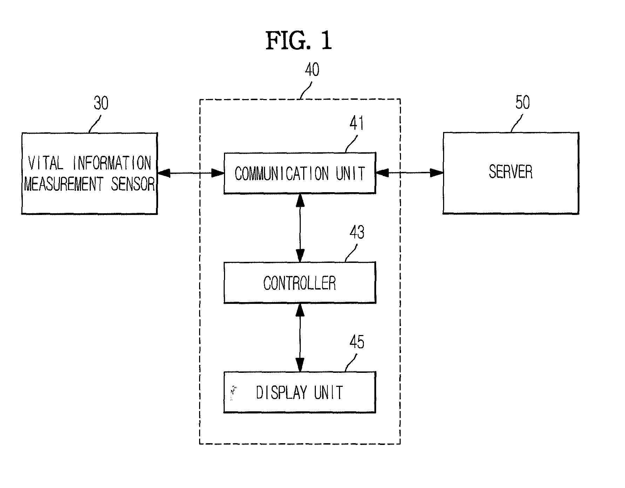

FIG. 1 is a block diagram illustrating an example of a remote healthcare system including a gateway. As is shown in the example illustrated in FIG. 1, the healthcare system example includes a vital information measurement sensor 30 configured to measure vital information of a user, a server 50 including a user database, and a gateway 40 configured to exchange information with the vital information measurement sensor 30 or the server 50.

The vital information measurement sensor 30 is a measurement device to measure the vital information of the user. The vital information measurement sensor 30 may include, but is not limited to, a blood pressure gauge, a blood sugar gauge, an electronic stethoscope, a peak flow meter, an exercise tester, a menu analyzer, a life pattern analyzer, a video camera, a body weight scale, a body composition analyzer, a medical imaging device, and a urine analyzer. When a user measures vital information using the vital information measurement sensor 30, the vital information measurement sensor 30 may transmit the measured vital information to the gateway 40.

The gateway 40 includes a communication unit 41 that may perform communication, a controller 43 that may generate a control signal according to information received from the communication unit 41, and a display unit 45 that may display information necessary for a patient according to the control signal of the controller 43. The gateway 40 may be a user terminal that enables the user to use the healthcare service through the remote healthcare system or other systems.

The gateway 40 may receive the healthcare service including healthcare items created based on user diagnostic information of medical professionals through a server of a hospital or a server of a healthcare center, and provide the healthcare service to the user, which is, for example, a patient. When the user measures a variety of vital information using the vital information measurement sensor 30 according to the healthcare service at home, the gateway 40 may receive the measured information from the vital information measurement sensor 30, and transmit the received information to the server 50, thereby enabling medical professionals to remotely treat the patient.

The user may be a user who uses the gateway 40. The user may be a patient who has a disease or a user who receives a remote healthcare service without having a disease using the gateway 40 for healthcare. Hereinafter, it is assumed that the patient is the user of the gateway 40.

The gateway 40 may be, but is not limited to being, a gateway including a display, a set top box type gateway including an IPTV or cable TV, a smart phone type gateway, a mobile phone type gateway, a WiBro terminal type gateway, a WiFi router type gateway, a PC type gateway including a tablet PC, and a gateway including a medical device.

The healthcare service may include patient care items suitable for patient healthcare, which are created based on patient diagnostic information of medical professionals. The healthcare service may further include, but is not limited to, a kind of vital information to be measured by a patient at home, an amount of times that the vital information has been measured, a measurement schedule of the vital information, a method of measuring the vital information including a method of using the vital information measurement sensor 30, a kind of a drug to be taken by the patient, an amount of the drug needs to be taken per unit time, when the patient needs to take the drug, a method of taking the drug, a menu of the patient that may include a recommended food list and a restricted food list, and an exercise plan configured to improve patient health.

The communication unit 41 of the gateway 40 performs communication with the vital information sensor 30 and the server 50. The communication unit 41 may receive the healthcare service from the server 50, and an instruction and recommendation necessary for patient healthcare from medical professionals through the server 50. Communication between the server 50 and the communication unit 41 of the gateway 40 may be performed using, but is not limited to, a WiFi communication method, a CDMA communication method, a wired/wireless local area network (LAN) communication method, a WiBro communication method, a 3G communication method, a 4G communication method, or a Public Switched Telephone Network (PSTN) communication method. The communication unit 41 may receive a variety of the vital information measured by the patient using the vital information measurement sensor 30 according to the healthcare service at home, and transmit the variety of the vital information to the server 50.

Communication between the communication unit 41 of the gateway 40 and the vital information measurement sensor 30 may be performed using, but is not limited to, a Bluetooth communication method, an infrared communication method, a WiFi communication method, a wired/wireless LAN communication method, a ZigBee communication method, a Serial communication method, or a Universal Serial Bus (USB) communication method.

The display unit 45 displays an image corresponding to a progress state of a healthcare service and an image corresponding to a health of a user. The display unit, for patient healthcare according to the healthcare service, may be configured to display information such as care information and patient information so as to enable the patient to recognize the care and patient information.

The display unit 45 may include a touchscreen. For example, the patient may directly touch various input buttons displayed on the display unit 45 so as to input external information. Further, a screen be changed according to such inputted external information. Although an example of a touchscreen for the display unit 45 is described, the display unit 45 is not limited thereto.

The controller 43 is configured to receive vital information from the vital information measurement sensor 30, determine the progress state of the healthcare service and the health of the user from the vital information, and display the image corresponding to the progress state of the healthcare service and the image corresponding to the health of user on the display unit 45. The controller may be a microcomputer that provides the patient with care information according to the healthcare service through the display unit 45 so as to administer patient healthcare according to the received healthcare service.

In addition, the controller 45 may also store personal information of a patient, current health of the patient, a healthcare service transmitted from the server 50, a progress state of the healthcare service, measured vital information of the patient transmitted from the vital information measurement sensor 30, a change in the health of the patient through analysis of the measured vital information of the patient, and an additional instruction or recommendation necessary to cure the patient transmitted from the server 50.

The server 50 includes a user database that may be used to store information about a user who uses the healthcare service. The server 50 including the user database may be further used to receive information necessary for user healthcare, such as vital information of the user, from the gateway 40 and transmit the information to medical professionals, thereby enabling the medical professionals to remotely administer healthcare.

The user database may include, but is not limited to, personal information of the user who uses the healthcare service, diagnostic information of the user created by the medical professionals, a healthcare item created based on the diagnostic information, measured vital information, and a result of analyzing the measured vital information.

Hereinafter, an example of a healthcare method using the above-described remote healthcare system example including the gateway 40 will be described.

The example of the healthcare method using the remote healthcare system including the gateway 40 includes accessing the healthcare system, acquiring vital information of a user who accesses the healthcare system, and displaying a progress state of a healthcare service and a health of the user as an image based on the acquired vital information.

The accessing of the healthcare system may include enabling the user to perform the accessing of the healthcare system. The vital information of the user may be acquired from the vital information measurement sensor 30. The acquired vital information of the user may be analyzed. The progress state of the healthcare service and the health of the user may be determined. The progress state and the health of the user may be provided to the user as an image.

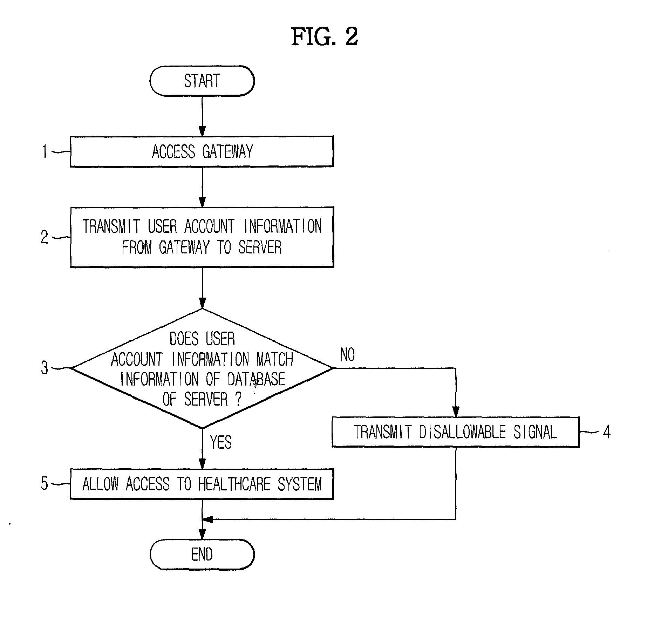

FIG. 2 is a flowchart illustrating an example of a process of accessing a healthcare system Referring to the example illustrated in FIG. 2, the user accesses the gateway 40 mounted at home in order to use the healthcare system (1). Access to the gateway 40 is performed by inputting user account information, including an ID and a password of the user, to the gateway 40.

When the user accesses the gateway 40, the gateway 40 transmits the user account information to the server 50 (2). The server 50 receives the user account information, including the ID and the password of the user, from the gateway 40, compares the received user account information with user account information stored in the user database of the server 50, and determines whether the received user account information matches the stored user account information (3).

The server 50 transmits a signal to the gateway 40 allowing user access to the healthcare system if the user account information received from the gateway 40 matches the user account information stored in the user database (5). If access to the healthcare system is allowed, the user may use the healthcare service provided by the healthcare system. The server 50 transmits a disallowable signal to the gateway 40 and prevents the user form accessing the healthcare system if the user account information received from the gateway 40 does not match the user account information stored in the user database (4).

When the user accesses the healthcare system, the gateway 40 receives the healthcare service from the server 50 and provides the healthcare service to the user. The user may perform a scheduled healthcare procedure according to various healthcare items included in the healthcare service. For example, the user may perform vital information measurement using the vital information measurement sensor 30 so as to determine his/her health.

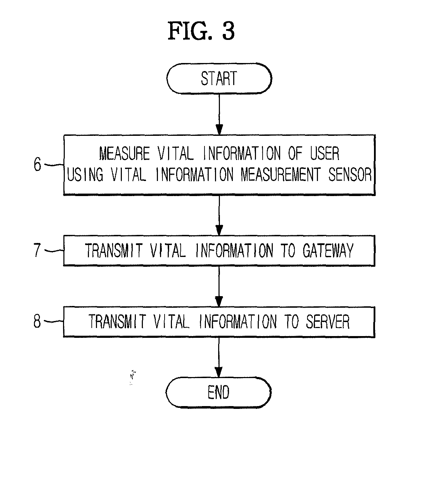

FIG. 3 is a flowchart illustrating an example of a process of acquiring vital information of a user. Referring to the example illustrated in FIG. 3, the user performs vital information measurement using the vital information measurement sensor 30 (6). The vital information measurement sensor 30 transmits the measured vital information to the gateway 40 upon completion of vital information measurement (7). The gateway 40 stores the received vital information of the user and transmits the vital information to the server 50 (8). The server 50 may store the vital information of the user received from the gateway 40 in the user database and update the information already stored in the user database. The gateway 40 may then analyze the vital information of the user, determine the progress state of the healthcare service and the health of the user according to the progress of the healthcare service, and notify the user of the progress state of the healthcare service and the health of the user.

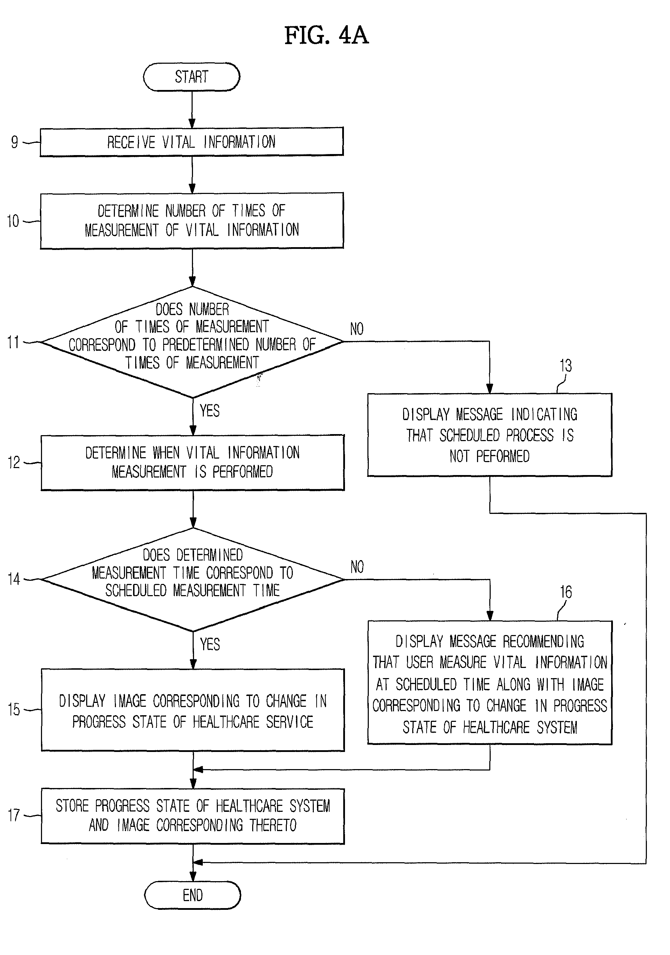

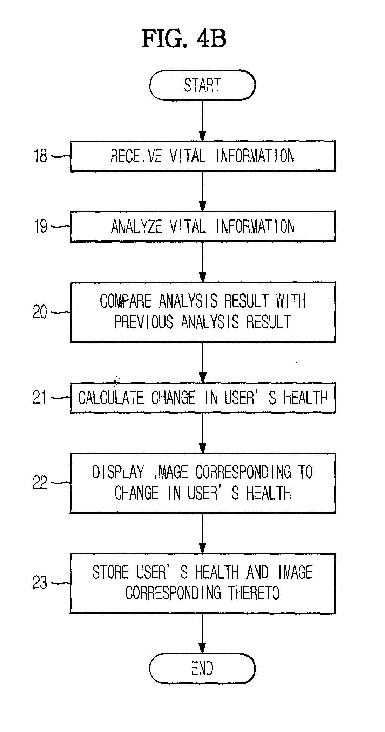

FIGS. 4A and 4B are flowcharts illustrating examples a process of displaying a progress state of a healthcare service and a health of a user as an image. Referring to the example illustrated in FIG. 4A, the gateway 40 receives the vital information of the user from the vital information measurement sensor 30 (9) and determines the total number of times that the vital information has been measured from the received vital information (10). Once the number of times of measurement is determined, a determination is made as to whether the number of times of measurement corresponds to the number of times of measurement predetermined in the healthcare service (11).

If it is determined that the number of times of measurement is less than or greater than the predetermined number of times of measurement, a message is displayed on the display unit 45 of the gateway 40 indicating that a scheduled process of the healthcare service is not performed (13). Due to this occurrence, since the scheduled process of the healthcare service is not performed, the change in the image corresponding to the change in the progress state is not performed.

If it is determined that the number of times of measurement is equal to the predetermined number of times of measurement, it is determined that the scheduled process is performed and a time when the vital information is measured is determined (12). A determination as to whether the determined measurement time corresponds to a measurement time scheduled in the healthcare service is then made (14).

If it is determined that the determined measurement time does not match the scheduled measurement time, a message recommending that the user measure the vital information at the scheduled time is displayed on the display unit 45 while displaying the image corresponding to the change in progress state of the healthcare service on the display unit 45 (16). If it is determined that the determined measurement time matches the scheduled measurement time, the image corresponding to the change in the progress state of the healthcare service is displayed on the display unit 45 (15).

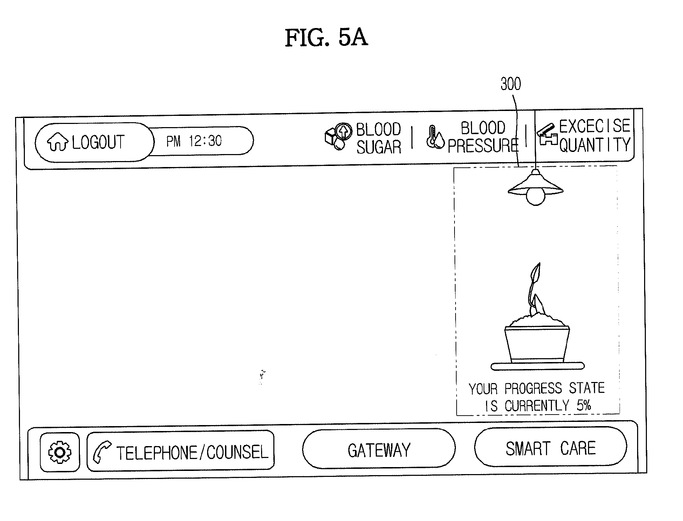

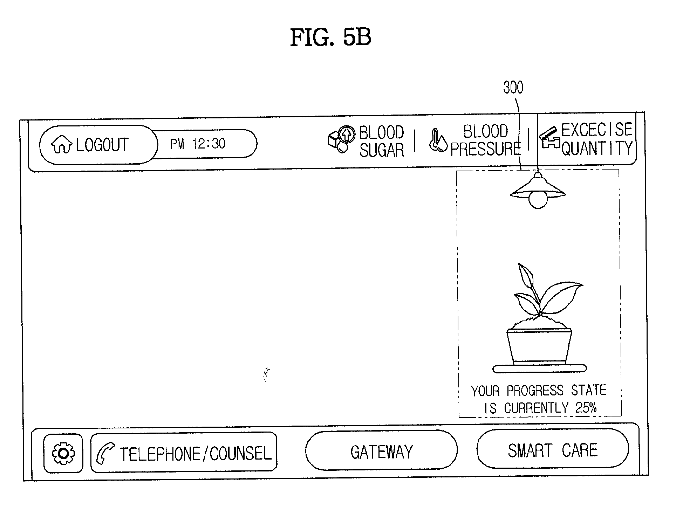

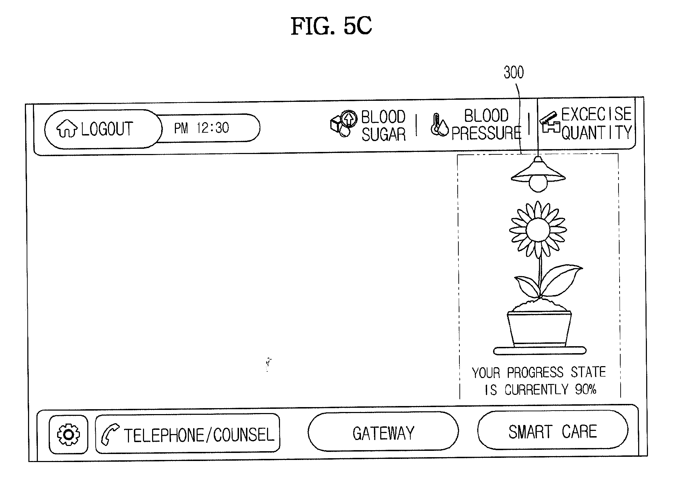

In order to enable the user to more accurately determine how much the healthcare service has progressed, a message explaining the progress rate of the healthcare service or the image displayed on the display unit 45 may be displayed along with the image. For example, a message indicating the progress rate of the healthcare service may be displayed. This example message may resemble the following recitation: "Your progress state is currently 5%". Alternatively, if the image displayed on the display unit 45 is a sprout in correspondence with the change in the progress state of the healthcare service, a message, such as "The current progress state is a sprout state", may be displayed along with a sprout image (see FIG. 5A). If the above-described process is performed by the progress state scheduled by a patient care module, the image corresponding to the change in the progress state of the healthcare service is gradually changed from the sprout image to an image of a flower in full bloom (FIGS. 5A to 5C). The sprout image is an example, and the image displayed is not limited thereto.

The change in the progress state of the healthcare service may be displayed using an avatar image of a user. The avatar image refers to various images including a photo of a user or a character image including features of a user. The image corresponding to the change in the progress state of the healthcare service is displayed on the display unit 45 by selecting the image, for example, the sprout image, corresponding to the current progress state of the healthcare service among a plurality of images corresponding to the change in the progress state of the healthcare service, for example, a plurality of images progressing from a sprout state to a flower in full bloom.

If the image corresponding to the change in the progress state of the healthcare service is displayed on the display unit 45, the progress state of the healthcare service and the image displayed on the display unit 45 in correspondence therewith are stored (17).

When the user accesses the gateway 40 and repeats the above-described process in the future, a determination is made as to whether a scheduled process is performed through comparison with the stored progress state of the healthcare service. If the scheduled process is performed, the image is changed from the stored image according to the performed process and the changed image is displayed on the display unit 45. If the image corresponding to the change in the progress state of the healthcare service is displayed on the display unit 45, the user may determine the current progress state of the healthcare service, may feel the necessity of healthcare, and become motivated to continue to use the healthcare service by viewing the changes in the image.

Referring to the example illustrated in FIG. 4B, the gateway 40 receives the vital information of the user from the vital information measurement sensor 30 (18), and analyzes the received vital information so as to calculate the result (19). The analysis result is compared with a previously stored analysis result (20) and a change in the health of the user is calculated (21). The change in the health of the user may be calculated by comparing and analyzing measured vital information such as a blood pressure level, a blood sugar level, a urine analysis result, a respiration analysis result, a blood analysis result, and a body fat analysis result with the previous information.

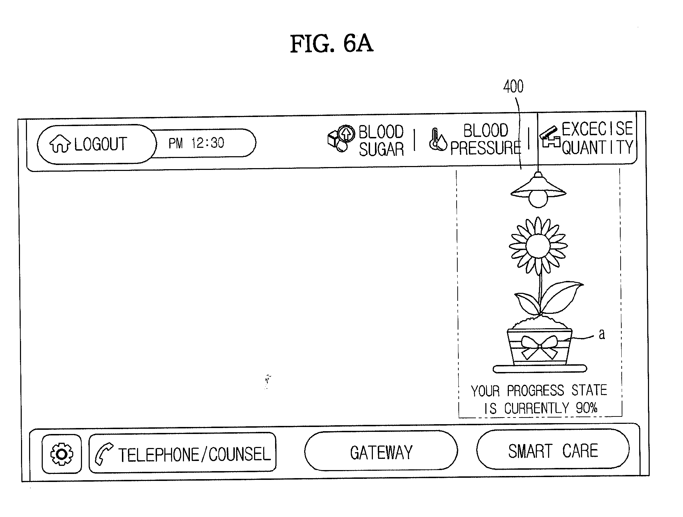

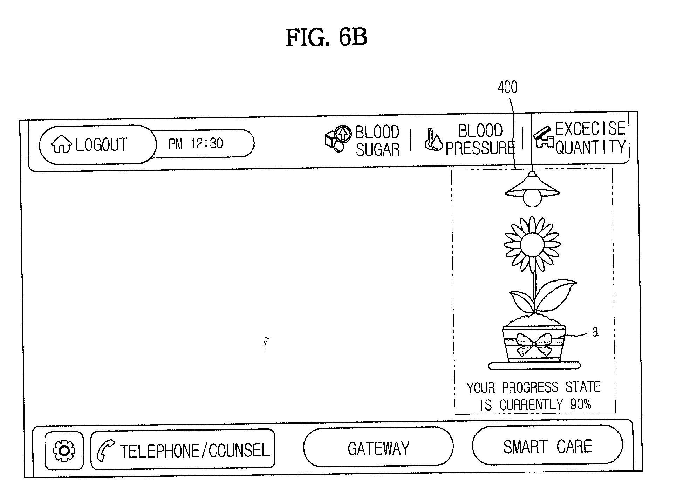

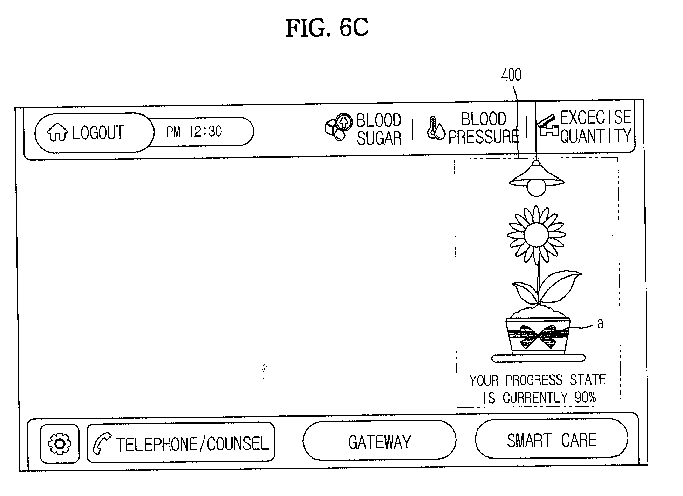

If the change in the health of the user is calculated, the image corresponding thereto is displayed on the display unit 45 (22). The change in the health of the user may be displayed by adding a ribbon a to an image corresponding to the change in the progress state of the healthcare service (i.e., a flowerpot), and changing the color of the ribbon a, as shown in FIGS. 6A to 6C. For example, as the health of the user improves, the color of the ribbon image is gradually changed from red to green so as to visually represent the improvement in the health of the user. The ribbon image is an example, and the image displayed is not limited thereto. The change in the health of the user may be displayed by displaying the avatar, the health condition of whom is improved, using the avatar image of the user. The avatar image refers to various images including a photo of a user or a character image including features of a user.

An achievement ratio indicating how much the current health condition has progressed toward a target health condition that is previously stored in the healthcare service or a change ratio indicating how much the current health condition has improved as compared to the previous health condition which is previously stored and a message explaining the image displayed on the display unit 45 may be displayed along with the image.

The target health condition may be previously stored in the healthcare service as a target value of the measured vital information. The achievement ratio is a ratio of a vital information measurement value indicating the current health condition to the target value of the measured vital information recorded in the healthcare service, and the change ratio is a ratio of a vital information measurement value indicating the current health condition to a previous vital information measurement value.

For example, a message, such as "The current health condition represents an achievement ratio of 70%" or "The current health condition has improved by 10%", indicating the achievement ratio or the change ratio of the health condition may be displayed along with the image. Alternatively, if the image displayed on the display unit 45 is a red ribbon in correspondence with the change in user's health, a message such as "The current health condition is a red ribbon state" may be displayed along with the image. If the health of the user continues to improve by the above-described process, the image corresponding to the change in the health of the user may gradually change from the red ribbon image to a green ribbon image.

As described above, the image corresponding to the change in the health of the user may be displayed on the display unit 45 by selecting an image corresponding to the current state of the health of the user. This may include, for example, a red ribbon image from among a plurality of images corresponding to the change in the health of the user, which may be, for example, a plurality of images changed from a red ribbon to a green ribbon and displaying the selected image on the display unit 45.

If an image corresponding to the change in the health of the user is displayed on the display unit 45, the health of the user and the image displayed on the display unit 45 in correspondence therewith are stored (23). When the user accesses the gateway 40 and repeats the above-described process in the future, a determination as to how much the health of the user has improved may be made by comparison with the stored health condition of the user, and the image changed according to the improvement of the health condition may be displayed on the display unit 45. If the image corresponding to the change in health of the user is displayed on the display unit 45, the user may determine his/her health, feel the necessity of healthcare, and become motivated to use the healthcare service by viewing the state in which the image is gradually changed.

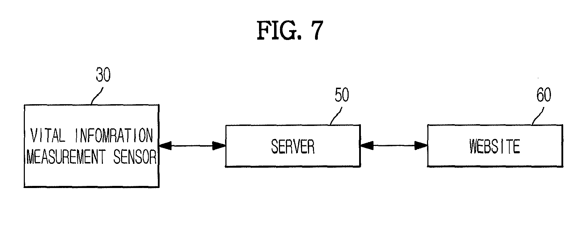

FIG. 7 is a block diagram illustrating an example of a remote healthcare system including a website 60. A description of the same parts as the remote healthcare system including the gateway 40 of FIG. 1 will be omitted. As shown in the example illustrated in FIG. 7, the healthcare system example includes a vital information measurement sensor 30, a server 50 including a user database, and a website 60 that may exchange information with the server 50.

The website 60 is configured to display a progress state of a healthcare service and a health of a user based on the vital information of the user. The website 60 may receive a healthcare service, including a healthcare item created based on diagnostic information of medical professionals, through a server of a hospital or a server of a healthcare center, and provide the healthcare service to a user, such as, for example, a patient. The website 60 may enable the user to use the healthcare service anywhere that the Internet is available without the need for a separate terminal.

Hereinafter, the healthcare method using the remote healthcare system example including the website 60 will be described. A description of the same portions as the healthcare method using the remote healthcare system including the gateway 40 will be omitted.

The healthcare method using the remote healthcare system example including the website 60 includes accessing the healthcare system, acquiring vital information of a user who accesses the healthcare system, and displaying a progress state and a health of a user as an image based on the acquired vital information.

The accessing of the healthcare system may include enabling the user to perform the accessing of the healthcare system. The vital information of the user may be acquired from the vital information measurement sensor 30. The acquired vital information of the user may be analyzed. The progress state of the healthcare service and the health of the user may be determined. The progress state and the health of the user may be provided to the user as an image.

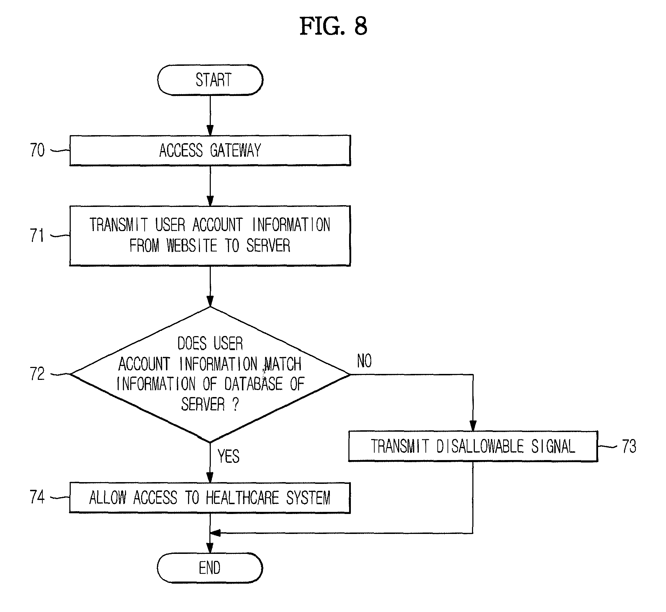

FIG. 8 is a flowchart illustrating an example of a process of accessing a healthcare system including a website 60. Referring to the example illustrated in FIG. 8, a user accesses the website 60 in order to access the healthcare system (70). Access to the website 60 may be performed by inputting user account information, including a user ID and password, to the website 60.

When the user accesses the website 60, the website 60 transmits the user account information to the server 50 (71). The server 50 receives the user account information, including the ID and the password of the user, from the website 60, compares the received user account information with user account information stored in the user database of the server 50, and determines whether the received user account information matches the stored user account information (72).

The server 50 transmits a signal to the website 60 allowing the user to access the healthcare system if the user account information received from the website 60 matches the user account information stored in the user database (74). If access to the healthcare system is allowed, the user may use the healthcare service provided by the healthcare system. The server 50 transmits a disallowable signal to the website 60 and does not allow access of the user to the healthcare system if the user account information received from the website 60 does not match the user account information stored in the user database (73).

When the user accesses the healthcare system, the website 60 receives the healthcare service from the server 50 and provides the healthcare service to the user. The user may perform a scheduled healthcare procedure according to various healthcare items included in the healthcare service. For example, the user may perform vital information measurement using the vital information measurement sensor 30 so as to determine his/her health.

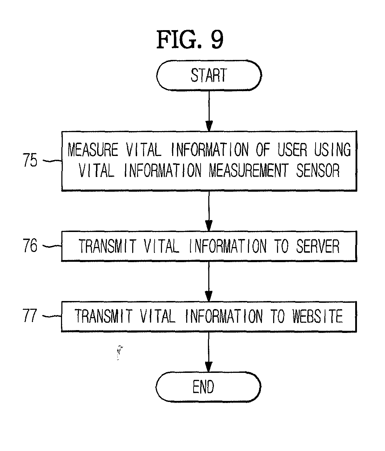

FIG. 9 is a flowchart illustrating another example of a process of acquiring vital information of a user. Referring to the example illustrated in FIG. 9, the user measures a scheduled vital information measurement procedure of the healthcare service using the vital information measurement sensor 30 (75). The vital information measurement sensor 30 transmits the measured vital information to the server 50 (76) once vital information measurement is completed. The server 50 may store the vital information of the user received from the vital information measurement sensor 30 in the user database and update the existing information. The server 50 transmits the received vital information of the user to the website 60 (77).

The website 60 may then analyze the vital information of the user so as to determine whether the healthcare service has progressed according to schedule and how the health of the user has changed in the course of the healthcare service and provide the determined result to the user.

The process of displaying the progress state of the healthcare service and the health of the user as the image is similar to the process shown in FIGS. 4A to 4B except that the website 60 is used instead of the gateway 40. In this example, the website 60 receives the vital information of the user from the server 50, and, thus, a description thereof will be omitted.

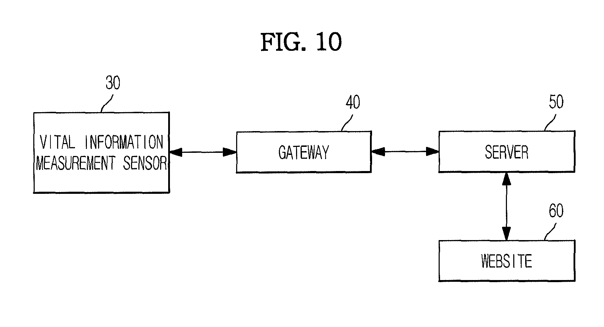

FIG. 10 is a block diagram illustrating another example of a remote healthcare system. As is illustrated in the example of FIG. 10, the healthcare system includes a vital information measurement sensor 30 configured to measure vital information of a user, a server 50 including a user database, and a website 60 configured to analyze the vital information of the user and display and provide a progress state of a healthcare service and a user's health to the user.

The healthcare system may further include a gateway 40 configured to receive the vital information of the user from the vital information measurement sensor 30 and transmit the vital information to the server 50. The website 60 may receive the vital information of the user from the server 50 or directly receive the vital information from the vital information measurement sensor 30 or the gateway 40. The user database may include, but is not limited to, personal information of a user who receives a healthcare service, diagnostic information of the user that is created by medical professionals, a healthcare item created based on the diagnostic information, measured vital information, and a result of analyzing the measured vital information. The remote healthcare system including both the website 60 and the gateway 40 may enable the user to conveniently select and use the website 60 and the gateway 40.

The healthcare method using the remote healthcare system including the website 60 and the gateway 40 is similar to the healthcare method using the remote healthcare system including the web 60 or the gateway 40 and a description thereof will thus be omitted.

According to the teachings above, there is provided a remote healthcare system and a healthcare method using the same in which, by displaying the image corresponding to the change in progress state of the healthcare service and the image corresponding to the change in health using the gateway or the website, the patient may easily determine the progress of the healthcare service and the change in the health of the patient, thereby motivating the patient to use the healthcare service. In addition, by collecting and using the vital information of the patient, a remote medical service may be provided. In addition, by providing a remote medical service through the website, the remote medical service may be provided more efficiently.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A healthcare method, comprising:
accessing a healthcare system;
acquiring vital information of a user who accesses the healthcare system; and
displaying a progress state of a healthcare service and a health of the user as an image based on the vital information.

2. The healthcare method according to claim 1, wherein the healthcare system includes:
a gateway that is a terminal used to access the healthcare system;
a vital information measurement sensor configured to:
measure the vital information of the user; and
transmit the vital information to the gateway; and
a server including a user database.

3. The healthcare method according to claim 1 or 2, wherein the accessing of the healthcare system comprises:
accessing the gateway;
transmitting user account information from the gateway to the server;
comparing the user account information with information stored in the user database at the server; and
allowing access to the healthcare system if the user account information matches the information stored in the user database.

4. The healthcare method according to claim 1, 2 or 3, wherein the acquiring of the vital information comprises:
collecting the vital information of the user using the vital information measurement sensor;
transmitting the vital information to the gateway; and
transmitting the vital information from the gateway to the server.

5. The healthcare method according to any of claims 1-4, wherein the displaying of the progress comprises:
determining the progress state of the healthcare service;
determining the health of the user;
displaying an image corresponding to the progress state of the healthcare service; and
displaying an image corresponding to the health of the user.

6. The healthcare method according to claim 5, wherein the determining of the progress state comprises:
determining a number of times of measurement of the vital information;
determining a time when the measurement of the vital information is performed;
determining whether the number of times of measurement of the vital information corresponds to a predetermined number of times of measurement of the healthcare service; and
determining whether the time when the vital information measurement is performed corresponds to a scheduled measurement time of the healthcare service.

7. The healthcare method according to claim 5 or 6, wherein the determining of the health comprises determining a change in the health of the user by comparing the vital information with previously measured vital information.

8. The healthcare method according to claim 5, 6 or 7, wherein:
the healthcare system includes:
a gateway that is a terminal used to access the healthcare system;
a vital information measurement sensor configured to:
measure the vital information of the user; and
transmit the vital information to the gateway; and
a server including a user database; and
the displaying of the image corresponding to the progress state of the healthcare service comprises:
selecting an image corresponding to a current progress state of the healthcare service from among a plurality of different images corresponding to a change in the progress state of the healthcare service;
displaying the selected image on the gateway; and displaying, on the gateway along with the image, an explanation of a progress rate of the healthcare service or the image.

9. The healthcare method according to any of claims 5-8, wherein:
the healthcare system includes:
a gateway that is a terminal used to access the healthcare system;
a vital information measurement sensor configured to:
measure the vital information of the user; and
transmit the vital information to the gateway; and
a server including a user database; and
the displaying of the image corresponding to the health of the user comprises:
selecting an image corresponding to a current health of the user from among a plurality of different images according to a change in the health of the user;
displaying the selected image on the gateway; and displaying, on the gateway along with the image, an explanation of a progress rate of the healthcare service or the image.

10. The healthcare method according to any of claims 5-9, wherein:
the healthcare system includes:
a server including a user database;
a vital information measurement sensor configured to:
measure the vital information of the user; and
transmit the vital information to the server; and
a website used to access the healthcare system; and
the displaying of the image corresponding to the progress state of the healthcare service comprises:
selecting an image corresponding to a current progress state of the healthcare service from among a plurality of different images corresponding to a change in the progress state of the healthcare service;
displaying the selected image on the website; and displaying, on the website along with the image, an explanation of a progress rate of the healthcare service or the image.

11. The healthcare method according to any of claims 5-10, wherein:
the healthcare system includes:
a server including a user database;
a vital information measurement sensor configured to:
measure the vital information of the user; and
transmit the vital information to the server; and
a website used to access the healthcare system; and
the displaying of the image corresponding to the health of the user comprises:
selecting an image corresponding to a current health of the user from among a plurality of different images according to a change in the health of the user;
displaying the selected image on the website; and displaying, on the website along with the image, an explanation of a progress rate of the healthcare service or the image.

12. A gateway, comprising:
a communication unit configured to perform communication with a vital information measurement sensor and a server;
a display unit configured to display an image corresponding to a progress state of a healthcare service and an image corresponding to a health of a user; and
a controller configured to:
receive vital information from the vital information measurement sensor;
determine the progress state of the healthcare service and the health of the user from the vital information; and
display the image corresponding to the progress state of the healthcare service and the image corresponding to the health of the user on the display unit.

13. The gateway according to claim 12, wherein the display unit operates in a touch screen manner.

14. The gateway according to claim 12 or 13, wherein the image corresponding to the progress state of the healthcare service comprises an image corresponding to a current progress state of the healthcare service among a plurality of different images according to a change in the progress state of the healthcare service, and wherein the image corresponding to the health of the user comprises an image corresponding to a current health of the user among a plurality of different images according to a change in the health of the user.

15. The gateway according to claim 12, 13 or 14, wherein the controller is further configured to display, on the display unit along with the image corresponding to the progress state of the healthcare service, an explanation of a progress rate of the healthcare service or the image, and wherein the controller is further configured to display, along with the image corresponding to the health of the user, an explanation of a change in the health of the user or the image on the display unit.
